# NEUE EUROPÄISCHE PATENTSCHRIFT

(11) **EP 1 158 013 B2**
(45) Veröffentlichungstag und Bekanntmachung des Hinweises auf die Entscheidung über den Einspruch: **11.03.2009**
(45) Hinweis auf die Patenterteilung: 16.03.2005
(21) Anmeldenummer: 01111131.7
(22) Anmeldetag: 09.05.2001
(51) Int. Cl.: C08G 18/78, C07C 273/18

(54) **Verfahren zur Herstellung von Polyisocyanaten mit Biuretstruktur**
Process for the preparation of polyisocyanates with biuret structure
Procédé de préparation de polyisocyanates à groupement biuret

(30) Priorität: 22.05.2000 DE 10025301
(43) Veröffentlichungstag der Anmeldung: 28.11.2001
(73) Patentinhaber: Bayer MaterialScience AG, 51368 Leverkusen (DE)
(72) Erfinder: Halpaap, Reinhard, Dr., 51519 Odenthal (DE); Mager, Dieter, 51373 Leverkusen (DE); Oeckl, Siegfied, Dr., 50169 Kerpen-Horrem (DE); Mertes, Harald, Dr., Bridgeville, PA 15017 (US)

(56) Entgegenhaltungen:
- EP-A- 0 012 970
- EP-A1- 0 716 080
- EP-A1- 0 918 809
- DE-A- 19 633 404
- US-A- 4 192 936

## Beschreibung

Die vorliegende Erfindung betrifft ein verbessertes Verfahren zur Herstellung von Polyisocyanaten mit Biuretstruktur durch kontinuierliche Umsetzung von überschüssigen Mengen an organischen Diisocyanaten mit ausschließlich aliphatisch und/oder cycloaliphatisch gebundenen Isocyanatgruppen mit organischen Diaminen mit ausschließlich aliphatisch und/oder cycloaliphatisch gebundenen primären Aminogruppen bei erhöhten Temperaturen in Gegenwart von Säuren. Die so hergestellten Polyisocyanate zeichnen sich durch hohe Stabilität und gute Verdünnbarkeit aus.

Die Herstellung von aliphatischen Polyisocyanaten mit Biuretstrukturen ist seit 1958 bekannt (DE-A 1 101 394). Weitere mögliche Herstellverfahren sind in einem Übersichtsartikel (Laas et al., J. prakt. Chem. 336, 1994, 185-200) beschrieben, die Vorund Nachteile der jeweiligen Verfahren diskutiert.

Man unterscheidet prinzipiell zwei Verfahrensgruppen: zum einen die sogenannten Wasserverfahren, bei denen die Diisocyanate mit Wasser zu Harnstoffen und nachfolgend Biureten umgesetzt werden, zum anderen die sogenannten Diisocyanat/Diamin-Verfahren, bei denen aus Isocyanat und Amin unmittelbar Harnstoff und nachfolgend Biuret hergestellt wird. Für beide Verfahren sind, wie im oben zitierten Übersichtsartikel (Laas et al.) ausgeführt, zahlreiche Varianten ausgearbeitet und beschrieben worden. Bei den beschriebenen Verfahren wird überwiegend mit Hexamethylendiisocyanat (HDI) und gegebenenfalls Hexamethylendiamin (HDA) zur Herstellung der technisch bedeutsamsten HDI-Biurete gearbeitet, wobei die zunächst erhaltenen in überschüssigem Diisocyanat gelöst vorliegenden Biurete durch Destillation und/oder Extraktion von überschüssigem Diisocyanat befreit und als monomerenarme Biuret-Polyisocyanate isoliert werden. Beide Verfahren sind ständig weiterentwickelt und optimiert worden.

Nach Wasserverfahren hergestellte Biuret-Polyisocyanate zeichnen sich in der Regel durch eine gute Monomerenstabilität, d. h. Stabilität gegenüber einer Rückspaltung in freie Diisocyanate, gute Verdünnbarkeiten, d. h. Stabilität verdünnter Lösungen bezüglich Trübungen und Ausfällungen bei Feuchteeinwirkung, sowie hervorragender Farbzahlen aufgrund der relativ milden Reaktionsbedingungen bei der Herstellung aus. Bei den Biuretisierungsreaktionen nach Wasserverfahren wird jedoch Prinzip-bedingt stets ein Teil der im Ausgangsgemisch enthaltenen Isocyanatgruppen durch Reaktion mit einem Biuretisierungsmittel in Aminogruppen umgewandelt. Da die so verbrauchten Isocyanatgruppen ursprünglich einmal durch Phosgenierung von Aminogruppen hergestellt wurden, erscheint diese Vorgehensweise zu aufwendig (wenig ökonomisch). Zudem entstehen bei diesen bekannten Verfahren gasförmige oder flüssige Nebenprodukte, wie zum Beispiel Kohlendioxid, Kohlenmonoxid, Kohlenoxidsulfid, Olefine oder Nitrile, die mit Ausnahme der beim Pivalinsäure-Verfahren anfallenden Anhydride nicht recyclisierbar sind und entsorgt werden müssen.

Bei den ausgearbeiteten Diisocyanat/Diamin-Verfahren zeigt sich der Vorteil der ökonomischen Herstellung mit keiner oder nur geringer Nebenproduktbildung, es werden keine durch Phosgenierung aus Aminogruppen hergestellte Isocyanatgruppen wieder in Aminogruppen, nachfolgend Harnstoff- und Biuretgruppen, zurückverwandelt. Diese Verfahren sind ebenfalls zu einem hohen Qualitätsstand ständig weiterentwickelt worden wie z. B. in der EP-A 277353 beschrieben. Ein gewisser Nachteil so hergestellter Biuret-Polyisocyanate besteht immer noch in einer leicht verminderten Monomerenstabilität und verminderter Verdünnungsstabilität, wobei bei Einstellung starker Verdünnungen (<40 % FK) leichte Trübungen und sogar Niederschläge auftreten können.

Schon seit längerer Zeit sind Biuretpolyisocyanate mit guter Monomerenstabilität bekannt, die mit Wasser als Biuretisierungsmittel unter saurer Katalye hergestellt werden. Neuere Arbeiten hierzu, die nach der Veröffentlichung von Laas et al., J. prakt. Chem. 1994 publiziert wurden, sind in EP-A 716080, WO 97/03044 und DE-A 19633404 beschrieben. In der EP-A 716080 wird ausgeführt, dass der Zusatz von OH-aciden Verbindungen, wie z.B. Dialkylphosphaten, die Bildung von unlöslichem Harnstoff bei der Biuretisierungsreaktion von aliphatischen Diisocyanaten mit Wasser unterdrückt. Nach der Lehre der DE-A 19633404 werden die Diisocyanate mit den Reaktanden in speziellen Mischelementen hoher Scherwirkung miteinander zur Reaktion gebracht. In den Beispielen der DE-A 19633404 werden auch sauer (Dialkylphosphat) katalysierte Umsetzungen genannt, sofern Wasser oder tert.-Butanol gegebenenfalls in Mischung mit Diamin als Reaktanden zum Einsatz gelangen. Beispiele dieser Anmeldung (Bsp. 1, Tabelle 1 und Bsp. 2, Tabelle 2) mit HDI und HDA wurden ohne saure Katalyse unter Einsatz der speziellen Mischelemente durchgeführt.

Die Aufgabe der vorliegenden Erfindung war es daher Polyisocyanate mit Biuretstruktur mit verbesserten Eigenschaften unter Beibehaltung des einfachen (wirtschaftlich günstigsten) Diisocyanat/Diamin-Verfahrens kontinuierlich herzustellen. Die Aufgabe der Erfindung war es insbesondere nach solch einem kontinuierlichen Verfahren Biuretpolyisocyanate mit verbesserter Monomerenstabilität sowie optimaler Verdünnbarkeit in organischen Lösungsmitteln ohne das Auftreten von Trübungen oder Ausfällungen herzustellen. Darüberhinaus sollten erfindungsgemäß hergestellte Polyisocyanate mit Biuretstruktur geringe Feuchteempfindlichkeit zeigen und niedrige Farbzahlen aufweisen. Auf die Notwendigkeit des Einsatzes spezieller Mischapparaturen zur Erzeugung hoher Scherkräfte sollte verzichtet werden können.

Wie jetzt überraschend gefunden wurde, ist es möglich, in einem kontinuierlichen Diisocyanat-Diamin Verfahren, wobei keine durch Phosgenierung von Aminogruppen hergestellten Isocyanatgruppen wieder in Aminogruppen zurückverwandelt werden, hochwertige Polyisocyanate mit Biuretstruktur auf Basis von organischen Diisocyanaten mit ausschließlich aliphatisch und/oder cycloaliphatisch gebundenen Isocyanatgruppen mit organischen Diaminen mit ausschließlich aliphatisch und/oder cycloaliphatisch gebundenen primären Aminogruppen mit verbesserten Eigenschaften und unter Verzicht auf spezielle Mischapparaturen herzustellen, wenn man die Ausgangsmaterialien bei Temperaturen oberhalb 170°C unter Zusatz von Säuren miteinander zur Reaktion bringt. Dieser Befund ist äußerst überraschend, da der Einsatz von OH-Säuren bei Diisocyanat/Diamin-Direktverfahren vermuten ließ, dass vor der eigentlichen Biuretisierung die OH-Säure durch das eingesetzte Amin neutralisiert und damit unwirksam wird. Außerdem war anzunehmen, dass die OH-Säure vom Isocyanat angegriffen und zumindest teilweise durch Umwandlung zum Anhydrid der Reaktion entzogen wird. Bei den vorgenannten Wasserverfahren ist dieses Unwirksamwerden nicht zu befürchten, da die Säure durch den Wasserzusatz immer wieder zurückgebildet werden kann.

Es war demnach ganz und gar nicht zu erwarten, dass die Dosierung von Säuren bei dem einfachen (wirtschaftlich interessanten) Diisocyanat/Diamin-Verfahren eine Reihe bedeutsamer Vorteile bringt:
- die Monomerenstabilität der erzeugten Biuretpolyisocyanate wird deutlich verbessert;
- die Empfindlichkeit der erzeugten Biuretpolyisocyanate gegenüber feuchten Lösemitteln wird entscheidend vermindert;
- die notwendige Reaktionstemperatur der Umsetzung von Diisocyanat mit Diamin kann ohne eine Verlängerung der Reaktionszeit und ohne das intermediäre Auftreten von Trübungen (Polyharnstoffe) abgesenkt werden, was eine deutliche Energieeinsparung zur Folge hat.

Gegenstand der Erfindung ist somit ein Diisocyanat-Diamin Verfahren zur kontinuierlichen Herstellung von Polyisocyanaten mit Biuretstruktur durch kontinuierliche Umsetzung von überschüssigen Mengen an organischen Diisocyanaten mit ausschließlich aliphatisch und/oder cycloaliphatisch gebundenen Isocyanatgruppen mit organischen Diaminen mit ausschließlich aliphatisch und/oder cycloaliphatisch gebundenen primären Aminogruppen bei Temperaturen oberhalb 170°C, dadurch gekennzeichnet, dass bei der Umsetzung Säuren zudosiert werden und keine durch Phosgenierung von Aminogruppen hergestellten Isocyanatgruppen wieder in Aminogruppen zurückverwandelt werden.

Ausgangsmaterialien für das erfindungsgemäße Verfahren sind organische Diisocyanate mit ausschließlich aliphatisch und/oder cycloaliphatisch gebundenen Isocyanatgruppen eines unter 300 liegenden Molekulargewichts. Beispiele derartiger Diisocyanate sind 1,4-Diisocyanatobutan, 1,6-Diisocyanatohexan (Hexamethylendiisocyanate, HDI), 1,6-Diisocyanato-2,2,4-trimethylhexan und/oder 1,6-Diisocyanato-2,4,4-trimethylhexan, 1,4- und/oder 1,5-Diisocyanatohexan, 2,6-Diisocyanatohexansäureethylester, 1,12-Diisocyanatododecan, 1,4-Diisocyanatocyclohexan, 2,4- und/oder 2,6-Diisocyanato-1-methylcyclohexan, 1-Isocyanato-3,3,5-trimethyl-5-isocyanatomethylcyclohexan (Isophorondiisocyanat, IPDI), 1,3- und/oder 1,4-Bis (isocyanatomethyl)cyclohexan, 4,4'-Diisocyanatodicyclohexylmethan oder 6-Isocyanatohexansäure-2-isocyanatoethylester. Beliebige Gemische derartiger Diisocyanate können ebenfalls verwendet werden. 1,6-Diisocyanatohexan ist besonders bevorzugt.

Weitere Ausgangsmaterialien für das erfindungsgemäße Verfahren sind organische Diamine mit ausschließlich aliphatisch und/oder cycloaliphatisch gebundenen primären Aminogruppen. Sie haben ein Molekulargewicht unter 300. Beispiele sind 1,2-Diaminoethan, 1,2-Diaminopropan, 1,3-Diaminopropan, 1,4-Diaminobutan, 1,6-Diaminohexan, 1,6-Diamino-2,2,4-trimethylhexan und/oder 1,6-Diamino-2,4,4-trimethylhexan, 1,4- und/oder 1,5-Diaminohexan, 2,4- und/oder 2,6-Diamino-1-methylcyclohexan, 1-Amino-3,3,5-trimethyl-5-aminomethylcyclohexan, 1,3- und/oder 1,4-Bis(aminomethyl)cyclohexan oder 4,4'-Diaminodicyclohexylmethan. Beliebige Gemische derartiger Diamine können ebenfalls eingesetzt werden. 1,6-Diaminohexan ist besonders bevorzugt.

Bei der Durchführung des erfindungsgemäßen Verfahrens werden die genannten Ausgangsdiisocyanate und Diamine in solchen Mengenverhältnissen zur Umsetzung gebracht, die einem Äquivalentverhältnis von Isocyanatgruppen zu Aminogruppen von mindestens 4:1, vorzugsweise von 4:1 bis 25:1 und insbesondere von 7:1 bis 20:1 entsprechen, wobei die primären Aminogruppen als monofünktionelle Gruppen in die Berechnung eingehen.

Als Katalysatoren kommen beim erfindungsgemäßen Verfahren beliebige Säuren, vorzugsweise Protonensäuren, mit einem pKs-Wert < 10 zum Einsatz. Bevorzugte saure Katalysatoren sind Phosphorsäure bzw. Phosphorsäureester, wie z. B. Methylphosphat, Ethylphosphat, n-Butylphosphat, n-Hexylphosphat, 2-Ethylhexylphosphat, Isooctylphosphat, n-Dodecylphosphat, Dimethylphosphat, Diethylphosphat, Di-npropylphosphat, Di-n-butylphosphat, Di-n-amylphosphat, Diisoamylphosphat, Di-ndecylphosphat, Diphenylphosphat oder Dibenzylphosphat, Sulfonsäuren, wie z.B. Methansulfonsäure, Ethansulfonsäure, Propansulfonsäure, 2- bzw. 4-Toluolsulfonsäure oder Naphthalin-1-sulfonsäure, oder auch Mono- und Dicarbonsäuren, wie z.B. Ameisensäure, Essigsäure, Propionsäure, Buttersäure, Pivalinsäure, Stearinsäure, Cyclohexancarbonsäure, Oxalsäure, Malonsäure, Bersteinsäure, Adipinsäure, Benzoesäure oder Phthalsäure. Besonders bevorzugt sind Dialkylphosphate der genannten Art. Ganz besonders bevorzugter Katalysator ist Di-n-butylphosphat.

Diese Säuren kommen beim erfindungsgemäßen Verfahren in Mengen von 0,01 bis 1,0 Gew.-%, vorzugsweise von 0,02 bis 0,5 Gew.-% und ganz besonders bevorzugt von 0,05 bis 0,5 Gew.-%, bezogen auf die Gesamtmenge an eingesetzten Ausgangsdiisocyanaten. Die Säuren können gelöst in einem geeigneten Lösungsmittel zugegeben werden. Vorzugsweise werden die Säuren in Substanz zugegeben.

Das erfindungsgemäße Verfahren wird vorzugsweise lösemittelfrei durchgeführt. Durchaus möglich ist jedoch auch die Mitverwendung geeigneter, unter den Reaktionsbedingungen inerter Lösungsmittel. Geeignet sind zum Beispiel Hexan, Ethylacetat, Butylacetat, 1-Methoxypropyl-2-acetat, Propylenglykoldiacetat, 2-Butanon, 4-Methyl-2-pentanon, Cyclohexanon, Toluol, Xylol, höher substituierte Aromaten, wie sie beispielsweise unter den Bezeichnungen Solventnaphtha® , Solvesso®, Isopar® , Nappar® (Deutsche EXXON CHEMICAL GmbH, Köln) und Shellsol® (Deutsche Shell Chemie GmbH, Eschborn) im Handel sind, oder Trialkylphosphate, wie z.B. Trimethylphosphat, sowie beliebige Gemische solcher Lösemittel.

Zur Durchführung des erfindungsgemäßen Verfahrens werden die Ausgangsmaterialien sofort nach ihrer Durchmischung bei einer Temperatur von oberhalb 170°C, vorzugsweise von oberhalb 200°C, insbesondere von 230 bis 320°C miteinander zur Reaktion gebracht. Diese hohen Reaktionstemperaturen zu Beginn der erfindungsgemäßen Umsetzung können durch Vorerhitzen des Diisocyanats auf Temperaturen von oberhalb 160°C, vorzugsweise von oberhalb 220°C erreicht werden. Im Falle der Verwendung eines hohen Diisocyanatüberschusses erübrigt sich dann oftmals ein Vorerhitzen der Diamine, im allgemeinen werden jedoch auch diese auf ca. 50 bis 200°C vorerhitzt. In der Regel kann davon ausgegangen werden, dass sich das Reaktionsgemisch auch in Abwesenheit einer Beheizung des Mischgefäßes unmittelbar nach seiner Herstellung durch Vermischen der Ausgangsmaterialien wegen der starken Wärmetönung der spontan ablaufenden Reaktion auf eine Temperatur erhitzt, die ca. 20 bis 70°C oberhalb der Temperatur liegt, die aufgrund des Aufheizens der Ausgangsmaterialien ohne Einbeziehung der Wärmetönung erwartet werden kann. Die zur Sicherstellung der erfindungswesentlichen hohen Temperaturen erforderlichen Aufheiztemperaturen der Ausgangsmaterialien können in guter Näherung aus der spezifischen Wärme der Ausgangsmaterialien (ca. 0,5 kcal/kg K) sowie der Reaktionsenthalpie der Reaktion (ca. 35 kcal/mol) abgeschätzt und auch erforderlichenfalls durch einen orientierenden Vorversuch ermittelt werden.

Die in jedem Fall erforderliche Aufheizung der Diisocyanate muß wegen der bekannten Temperaturempfindlichkeit dieser Verbindungen innerhalb eines möglichst kurzen Zeitraums bewerkstelligt werden, vorzugsweise innerhalb eines Zeitraums von weniger als 30 Sekunden. Dies gelingt durch Verwendung von entsprechenden Wärmeaustauschaggregaten des Standes der Technik. Die Wärmetauscher können z.B. als Röhren, Bündel oder Plattenwärmeaustauscher ausgelegt sein. Sie können mit einem flüssigen Heizmedium, mit gespanntem Dampf oder mit direkter elektrischer Heizung betrieben werden. Besonders bevorzugt ist die Verwendung von solchen Wärmetauschern, die den Aufheizvorgang der Ausgangsdiisocyanate innerhalb eines Zeitraums von weniger als 3 Sekunden gestatten.

Die kontinuierlichen Ströme der Reaktionspartner werden nach der beschriebenen Vorheizung in einer Mischkammer vereinigt An die Leistungsfähigkeit der Mischkammer hinsichtlich einer intensiven Vermischung der Komponenten werden bei dem erfindungsgemäßen Verfahren keine besonderen Anforderungen gestellt. Beliebige statische oder dynamische Aggregate des Standes der Technik können zum Einsatz kommen. Im allgemeinen ist als Mischkammer ein einfaches Reaktionsrohr ohne jegliche Einbauten, an dessen einem Ende die Reaktionskomponenten im Gleichstrom eingebracht werden, vollkommen ausreichend und wird auch vorzugsweise verwendet.

Die Eintrittsstellen der Komponenten und Austrittsstellen des Reaktionsgemisches sind vorzugsweise in Form von Lochblenden oder Düsen ausgebildet, damit die Zudosierung unter Überdruck erfolgen kann. Dadurch kann gewährleistet werden, dass das Reaktionsgemisch nicht in die Zuleitungen von Diisocyanat und Diamin gelangen kann. Hierfür werden die Querschnitte so gewählt, dass sich auf den Zuleitungen jeweils ein Druck von 1,5 bis 100 bar, vorzugsweise von 1,5 bis 40 bar aufbaut. Form und Anordnung der Düsen und/oder Lochblenden sowie hoher Druck sind für das Verfahren nicht erfindungswesentlich, da an die Mischleistung keine hohen Anforderungen gestellt werden.

Die Dosierung der Säuren erfolgt zweckmäßigerweise im Bereich der Mischkammer, vorzugsweise in die Isocyanatkomponente unmittelbar vor der Dosierung des Amins. Zur Eindosierung der Säuren können übliche Pumpen des Standes der Technik, wie z. B. Kolben- oder Kolbenmembranpumpen, verwendet werden. Es ist lediglich erforderlich, dass der Eindosierdruck höher als der Mischkammerdruck ist.

Nach Durchlaufen der Mischkammer und der gegebenenfalls der Mischkammer nachgeschalteten Verweilzeitstrecke wird das Reaktionsgemisch kontinuierlich durch geeignete Wärmeaustauscher innerhalb von höchstens 10 Minuten, vorzugsweise höchstens 5 Minuten, stetig oder stufenweise auf eine Temperatur innerhalb des Temperaturbereichs von 80 bis 220°C, vorzugsweise 120 bis 200°C, abgekühlt und in diesen Temperaturbereichen mittels eines geeigneten Nachreaktors vorzugsweise während eines Zeitraumes von höchstens 5 Stunden, vorzugsweise höchstens 2 Stunden, insbesondere bis zu 30 Minuten einer thermischen Nachbehandlung unterzogen. Wesentlich ist hierbei vor allem, dass das Reaktionsgemisch den Maximaltemperaturen von über 170°C, vorzugsweise über 200°C, besonders bevorzugt über 230°C nur kurze Zeit ausgesetzt wird. Die Verweilzeiten in diesem Maximaltemperaturbereich sollten im Minuten- bis Sekundenbereich, vorzugsweise unter 60 Sekunden liegen. Die Dauer der thermischen Nachbehandlung kann innerhalb weiter Grenzen schwanken. Im allgemeinen ist bei niedrigen Temperaturen innerhalb der zuletzt genannten Bereiche eine vergleichsweise lange, bei hohen Temperaturen eine vergleichsweise kurze thermische Nachbehandlung erforderlich.

Die thermische Nachbehandlung kann beispielsweise in kaskadenförmig angeordneten Reaktoren oder in kontinuierlich durchflossenen Rührkesseln durchgeführt werden.

Im Anschluß an die thermische Nachbehandlung liegt als Reaktionsprodukt eine Lösung von Biuretgruppen-aufweisenden Polyisocyanaten in überschüssigem Ausgangsdiisocyanat und gegebenenfalls mitverwendeten Lösungsmitteln vor, falls diese nicht bereits während der Umsetzung abdestilliert worden sind. Das vorliegende Gemisch wird anschließend im allgemeinen durch Destillation im Hochvakuum, bevorzugt im Dünnschichtverdampfer, beispielsweise bei einer Temperatur von 100 bis 200°C, vorzugsweise von 120 bis 180°C, von flüchtigen Bestandteilen (überschüssigen monomeren Diisocyanaten und gegebenenfalls mitverwendeten Lösungsmitteln) befreit.

In einer weiteren Ausführungsform des erfindungsgemäßen Verfahrens werden die genannten flüchtigen Bestandteile durch Extraktion mit geeigneten gegenüber Isocyanatgruppen inerten Lösungsmitteln, beispielsweise aliphatischen oder cycloaliphatischen Kohlenwasserstoffen wie Pentan, Hexan, Heptan, Cyclopentan oder Cyclohexan vom Reaktionsprodukt abgetrennt.

Auf diese Weise erhält man hochwertige Polyisocyanate mit Biuretstruktur, die einen Gehalt an überschüssigem Ausgangsdiisocyanat von maximal 0,5 Gew.-%, vorzugsweise von maximal 0,3 Gew.-%, aufweisen.

Die nach dem erfindungsgemäßen Verfahren hergestellten, Biuretgruppen aufweisenden Polyisocyanate, insbesondere jene, die unter ausschließlicher Verwendung von 1,6-Diisocyanatohexan und 1,6-Diaminohexan als Ausgangsmaterialien hergestellt worden sind, stellen wertvolle Ausgangsmaterialien für die Herstellung von Zweikomponenten Polyurethanlacken dar. Die erfindungsgemäß hergestellten Produkte weisen ebenso wie die bisher bekannten Biuretpolyisocyanate des Standes der Technik gute Farbzahlen und vergleichsweise niedrige Viskositäten auf, zeichnen sich gegenüber diesen aber durch eine erheblich verbesserte Monomerenstabilität, eine geringere Empfindlichkeit gegenüber Feuchtigkeit sowie eine deutlich verbesserte Verdünnbarkeit mit organischen Lösungsmitteln aus.

### Beispiele

In den nachfolgenden Beispielen beziehen sich alle Prozentangaben auf Gewichtsprozente.

### Beispiel 1 (Vergleich nach EP-A 277 353)

In einer Vesuchsapparatur zur kontinuierlichen Herstellung von Biuret-Polyisocyanaten wurden 667 Teile Hexamethylendiisocyanat (HDI) pro Stunde bei 250°C kontinuierlich durch eine Reaktionsmischkammer geleitet. In diese Mischkammer wurden nun 27 Teile Hexamethylendiamin (HDA) pro Stunde ebenfalls kontinuierlich eingespeist, wobei die Temperatur in der Mischkammer durch die Reaktionswärme auf 275°C anstieg. Nach Verlassen der Mischkammer wurde das Produkt innerhalb weniger Sekunden auf 180°C abgekühlt und bei 180 - 140°C noch einige Minuten nachgetempert. Anschließend wurde aus der so erhaltene Rohware mit Hilfe der üblichen Dünnschicht-Destillationstechnik überschüssiges HDI abgetrennt. Es wurde ein als Lackhärter geeignetes Biuret-Polyisocyanat mit folgenden Kenndaten erhalten:

| | |
|---|---|
| NCO | 22,0 % |
| Viskosität | 10 000 mPas (23°C) |

### Beispiel 2 (erfindungsgemäß)

Man verfährt zunächst mit den gleichen Mengen, wie in Beispiel 1 beschrieben, mit dem Unterschied, dass nun in die HDI-Zuleitung kurz vor der Mischkammer zusätzlich ein kontinuierlicher Strom von 1 Teil Di-n-butylphosphat (DBP) pro Stunde injiziert wird. Die Mischkammertemperatur stieg dadurch um 5°C auf 280°C an. Die so erhaltene Rohware wurde wie in Beispiel 1 beschrieben mit Hilfe der üblichen Dünnschicht-Destillationstechnik von überschüssigem HDI befreit, und es wurde ein Biuret-Polyisocyanat **2a** mit folgenden Kenndaten erhalten:

| | |
|---|---|
| NCO | 21,8% |
| Viskosität | 11 100 mPas (23°C) |

Die im Vergleich zum Polyisocyanat aus Beispiel 1 ungünstigere höhere Viskosität des erfindungsgemäß hergestellten Produkts kann durch Absenken der HDI-Einlauftemperatur von 250°C auf 230°C sehr leicht an die Kenndaten des unkatalysiert hergestellten Biuret-Polyisocyanats angepaßt werden. Durch diese Maßnahme erhält man ein Produkt **2b** mit den folgenden Kenndaten:

| | |
|---|---|
| NCO | 22,1 % |
| Viskosität | 9 520 mPas (23°C) |

Das erfindungsgemäße Verfahren ermöglicht somit im Vergleich zum unkatalysierten Herstellverfahren eine Absenkung der Reaktionstemperatur, woraus sich eine erhebliche Energieeinsparung beim Aufheizen des HDI ergibt.

### Beispiel 3 (Vergleich nach EP-A 277 353)

Unmittelbar im Anschluß an die Herstellung der erfindungsgemäßen Biuret-Polyisocyanate **2a** und **2b** wird unter Beibehaltung der kontinuierlichen Verfahrensweise mit konstanten Mengenströmen an HDI und HDA die DBP-Dosierung beendet und die HDI-Einlauftemperatur wieder auf 250°C erhöht. Nach Aufarbeitung der Rohware durch Dünnschichtdestillation erhält man ein Produkt mit den folgenden Kenndaten:

| | |
|---|---|
| NCO | 22,1 % |
| Viskosität | 10 200 mPas (23°C) |

### Beispiel 4 (Prüfung der Lagerstabilität)

Zur Überprüfung der Lagerstabilität wurden Proben der Biuret-Polyisocyanate aus Beispiel 1 bis 3 für 4 Wochen bei 35°C gelagert. Die nachstehende Tabelle zeigt die Gehalte an monomerem HDI, die jeweils vor Beginn der Wärmelagerung sowie nach 2 bzw. 4 Wochen gemessen wurden. Der Vergleich zeigt, dass die erfindungsgemäß unter Zusatz von DBP hergestellten Proben eine deutlich geringere Rückspalttendenz aufweisen.

| Probe | HDI [%] | | |
|---|---|---|---|
| | Anfangswert | nach 2 Wochen 35°C | nach 4 Wochen 35°C |
| Beispiel **1** | 0,31 | 0,43 | 0,50 |
| Beispiel **2b** / Probe 1 | 0,21 | 0,26 | 0,24 |
| Beispiel **2b** / Probe 2 | 0,23 | 0,33 | 0,34 |
| Beispiel **2b** / Probe 3 | 0,21 | 0,32 | 0,29 |
| Beispiel **3** | 0,20 | 0,39 | 0,44 |

### Beispiel 5 (Prüfung der Verdünnbarkeit)

Die Biuretpolyisocyanate aus Beispiel **1** (Vergleich) und Beispiel **2b** (erfindungsgemäß) werden jeweils mit Butylacetat zu 75 % Festkörper (FK) aufweisenden Polyisocyanatlösungen verdünnt. Das verwendete Butylacetat wurde vorher ebenso wie alle nachfolgend verwendeten Lösemittel mit Molekularsieb (Typ: Baylith® SV 133; Bayer AG, Leverkusen) entwässert.

Die Biuretlösungen wurden mit den nachfolgend aufgeführten Lösungsmitteln oder Lösungsmittelgemischen durch Verdünnen jeweils auf FK-Gehalte von 35, 30, 25 und 20 % eingestellt. Die Lösungen wurden in verschlossenen Flaschen 28 Tage bei 50°C oder 4 Monate bei Raumtemperatur (RT) gelagert, anschließend die Verdünnungsstabilität visuell begutachtet. Bei der Beurteilung wird unterschieden zwischen 0 für unverändert, 1 für eine leichte Trübung bzw. Bodensatz und 2 für eine starke Trübung bzw. Bodensatz.

Die nachfolgende Tabelle zeigt die Ergebnisse der 50°C-Lagerung (28 Tage).

| Polyisocyanat aus | Beispiel **1** (Vergleich) | | | | Beispiel **2b** (erfindungsgemäß) | | | |
|---|---|---|---|---|---|---|---|---|
| FK-Gehalt: | 35 % | 30% | 25 % | 20% | 35 % | 30 % | 25 % | 20 % |
| MPA | 1 | 2 | 2 | 2 | 0 | 0 | 0 | 0 |
| BA/SN (1:2) | 0 | 2 | 2 | 2 | 0 | 0 | 0 | 1 |
| X | 0 | 2 | 2 | 2 | 0 | 0 | 0 | 1 |
| BA | 0 | 0 | 1 | 2 | 0 | 0 | 0 | 1 |
| MPA/X (1:1) | 0 | 0 | 0 | 1 | 0 | 0 | 0 | 0 |
| EA | n.b. | 1 | n.b. | 2 | n.b. | 0 | n.b. | 1 |
| Abkürzungen: MPA = 1-Methoxypropyl-2-acetat BA = Butylacetat SN = Solventnaphtha® 100 X = Xylol EA = Ethylacetat n.b. = nicht bestimmt. | | | | | | | | |

Die nachfolgende Tabelle zeigt die Ergebnisse der RT-Lagerung (4 Monate).

| Polyisocyanat aus | Beispiel **1** (Vergleich) | | | | Beispiel **2b** (erfindungsgemäß) | | | |
|---|---|---|---|---|---|---|---|---|
| FK-Gehalt: | 35% | 30% | 25 % | 20 % | 35% | 30 % | 25 % | 20% |
| MPA | 2 | 2 | 2 | 2 | 1 | 0 | 0 | 0 |
| BA/SN(1:2) | 0 | 2 | 2 | 2 | 0 | 0 | 0 | 0 |
| X | 0 | 2 | 2 | 2 | 0 | 0 | 0 | 2 |
| BA | 1 | 2 | 2 | 2 | 1 | 1 | 1 | 1 |
| MPA/X (1:1) | 0 | 0 | 2 | 2 | 0 | 0 | 0 | 0 |
| EA | n.b. | 2 | n.b. | 2 | n.b. | 1 | n.b. | 1 |

### Beispiel 6 (erfindungsgemäß 6a und Vergleich 6b)

In einem 11 Vierhalskolben mit Talerrührer, Thermometer und Destillationskolonne wird bei Raumtemperatur unter leichtem Stickstoffstrom eine Mischung aus 302,4 g HDI, 157 g Cyclohexan und 0,45 g DBP vorgelegt. Unter gutem Rühren wird dann innerhalb weniger Sekunden eine maximal 30°C warme Lösung aus 11,6 g HDA und 157 g Cyclohexan zugegeben. Die Temperatur steigt nur wenig an, die entstehende Harnstoffemulsion bleibt gut rührbar. Dann wird auf 180°C aufgeheizt, dabei wird das Cyclohexan abdestilliert. Nach ca. 1,5 bis 2 Stunden ist der Harnstoff gelöst. Man rührt noch 4 Stunden bei 180°C nach. Anschließend wird die Rohlösung in einem handelsüblichen Kurzwegverdampfer bei einer Temperatur von 130°C und einem Druck von 0,1 mbar von monomerem Isocyanat befreit. Man erhält ein klares praktisch farbloses Biuret-Polyisocyanat 6a mit den folgenden Kenndaten:

| | |
|---|---|
| NCO | 21,6 % |
| Viskosität | 9 700 mPas (23°C) |

Zum Vergleich wird nach dem gleichen Verfahren jedoch ohne Zusatz von DBP ein Biuret-Polyisocyanat hergestellt. Das klare praktisch farblose Produkt **6b** weist die folgenden Kenndaten auf:

| | |
|---|---|
| NCO | 21,7 % |
| Viskosität | 9 500 mPas (23°C) |

Zur Überprüfung der Lagerstabilität wurden beide Biuret-Polyisocyanate bis zu 12 Wochen bei verschiedenen Temperaturen gelagert. Die nachstehende Tabelle zeigt die Ausgangsgehalte an monomerem HDI sowie die Werte, die nach Wärme- bzw. Raumtemperaturlagerung bestimmt wurden.

| Biuret-Polyisocyanat | HDI [%] | | | |
|---|---|---|---|---|
| | Start | 1 Woche 80°C | 6 Wochen 50°C | 12 Wochen RT |
| erfindungsgemäß **6a** | 0,18 | 0,58 | 0,48 | 0,48 |
| Vergleich **6b** | 0,21 | 0,88 | 0,84 | 0,85 |

### Beispiel 7 (erfindungsgemäß 7a und Vergleich 7b)

In einem 250 ml Vierhalskolben mit Talerrührer, Thermometer und Kühler werden bei 240°C unter leichtem Stickstoffstrom 134,4g HDI vorgelegt. Unter gutem Rühren gibt man innerhalb weniger Sekunden 0,34g DBP und sofort im Anschluß daran 4,6g ca. 80°C warmes HDA zu. Die Temperatur der Mischung steigt rasch auf ca. 255°C an. Der Harnstoff löst sich sofort auf. Man läßt auf 170°C abkühlen und rührt noch 30 Minuten nach. Die Rohlösung, die einen NCO-Gehalt von 41,0 % aufweist, wird anschließend durch Dünnschichtdestillation bei 130°C im Hochvacuum von monomerem HDI befreit. Man erhält ein klares, fast farbloses Biuret-Polyisocyanat **7a** mit folgenden Kenndaten:

| | |
|---|---|
| NCO | 21,6 % |
| Viskosität | 13 200 mPas (23°C) |

Zum Vergleich wird nach dem gleichen Verfahren jedoch ohne Zusatz von DBP ein Biuret-Polyisocyanat hergestellt. Nach Dünnschichtdestillation der Rohlösung (NCO-Gehalt 42,0%) erhält man ein klares, praktisch farbloses Produkt **7b** mit folgenden Kenndaten:

| | |
|---|---|
| NCO | 21,7 % |
| Viskosität | 12 500 mPas (23°C) |

Zur Überprüfung der Lagerstabilität wurden beide Biuret-Polyisocyanate 1 Woche bei 80°C gelagert. Die nachstehende Tabelle zeigt die Ausgangsgehalte an monomerem HDI sowie die Werte, die nach Wärme- bzw. Raumtemperaturlagerung bestimmt wurden.

| Biuret-Polyisocyanat | HDI [%] | |
|---|---|---|
| | Start | 1 Woche 80°C |
| erfindungsgemäß **7a** | 0,15 | 0,45 |
| Vergleich **7b** | 0,78 | 1,90 |

## Patentansprüche

1. *Diisocyanat-Diamin*-Verfahren zur kontinuierlichen Herstellung von Polyisocyanaten mit Biuretstruktur durch kontinuierliche Umsetzung von überschüssigen Mengen an organischen Diisocyanaten mit ausschließlich aliphatisch und/oder cycloaliphatisch gebundenen Isocyanatgruppen mit organischen Diaminen mit ausschließlich aliphatisch und /oder cycloaliphatisch gebundenen primären Aminogruppen bei Temperaturen oberhalb 170°C, **dadurch gekennzeichnet, dass** bei der Umsetzung Säuren zudosiert werden und keine durch Phosgenierung von Aminogruppen hergestellten Isocyanatgruppen wieder in Aminogruppen zurückverwandelt werden.

2. Verfahren gemäß Anspruch 1, **dadurch gekennzeichnet, dass** bei der Umsetzung Säuren zudosiert werden, ausgewählt aus der Gruppe der Phosphorsäuren, Sulfonsäuren und Carbonsäuren.

3. Verfahren gemäß Anspruch 1, **dadurch gekennzeichnet, dass** bei der Umsetzung Phosphorsäuredialkylester zudosiert werden.

4. Verfahren gemäß Anspruch 1, **dadurch gekennzeichnet, dass** die bei der Umsetzung des Diisocyanats mit dem. Diamin zudosierten Säuren in Mengen von 0,01 bis 1,0 Gew.-% bezogen auf eingesetztes Diisocyanat verwendet werden.

5. Verfahren zur kontinuierlichen Herstellung von Polyisocyanaten mit Biuretstruktur gemäß Anspruch 1, **dadurch gekennzeichnet, dass** die bei der Umsetzung des Diisocyanats mit dem Diamin verwendeten Säuren dem Diisocyanat unmittelbar vor Zudosierung des Diamins zugefügt werden.

6. Verfahren zur kontinuierlichen Herstellung von Polyisocyanaten mit Biuretstruktur gemäß Anspruch 1 durch kontinuierliche Umsetzung von überschüssigen Mengen an HDI mit HDA bei Temperaturen oberhalb 170°C, **dadurch gekennzeichnet, dass** bei der Umsetzung Säuren zudosiert werden.

7. Verfahren zur kontinuierlichen Herstellung von Polyisocyanaten mit Biuretstruktur gemäß Anspruch 1 durch kontinuierliche Umsetzung von überschüssigen Mengen an HDI mit HDA bei Temperaturen oberhalb 200°C, **dadurch gekennzeichnet, dass** bei der Umsetzung Säuren zudosiert werden.

8. Verfahren zur kontinuierlichen Herstellung von Polyisocyanaten mit Biuretstruktur durch kontinuierliche Umsetzung von überschüssigen Mengen an organischen Diisocyanaten mit ausschließlich aliphatisch und/oder cycloaliphatisch gebundenen Isocyanatgruppen mit organischen Diaminen mit ausschließlich aliphatisch und/oder cycloaliphatisch gebundenen primären Aminogruppen bei Temperaturen oberhalb 170°C in Gegenwart von Säuren, nach Anspruch 1 und Isolierung des Polyisocyanats nach der Umsetzung wobei die erhaltene Polyisocyanatlösung durch Extraktion oder Dünnschichtdestillation von überschüssigem Diisocyanat auf Gehalte < 0,5 Gew.-% monomeres Diisocyanat befreit wird.

## Claims

1. Diisocyanate/diamine process for the continuous preparation of polyisocyanates with a biuret structure by continuously reacting excess amounts of organic diisocyanates having exclusively aliphatically and/or cycloaliphatically bound isocyanate groups with organic diamines having exclusively aliphatically and/or cycloaliphatically bound primary amino groups at temperatures above 170°C, **characterized in that** acids are metered in during the reaction and no isocyanate groups prepared from amino groups by phosgenation are converted back into amino groups.

2. Process according to Claim 1, **characterized in that** during the reaction acids are metered in selected from the group consisting of phosphoric acids, sulfonic acids and carboxylic acids.

3. Process according to Claim 1, **characterized in that** during the reaction phosphoric acid dialkyl esters are metered in.

4. Process according to Claim 1, **characterized in that** the acids metered in during the reaction of the diisocyanate with the diamine are used in amounts from 0.01-1.0 wt. %, based on diisocyanate used.

5. Process for the continuous preparation of polyisocyanates with a biuret structure according to Claim 1, **characterized in that** the acids used during the reaction of the diisocyanate with the diamine are added to the diisocyanate immediately before the diamine is metered in.

6. Process for the continuous preparation of polyisocyanates with a biuret structure according to Claim 1 by continuously reacting excess amounts of HDI with HDA at temperatures above 170°C, **characterized in that** acids are metered in during the reaction.

7. Process for the continuous preparation of polyisocyanates with a biuret structure according to Claim 1 by continuously reacting excess amounts of HDI with HDA at temperatures above 200°C, **characterized in that** acids are metered in during the reaction.

8. Process for the continuous preparation of polyisocyanates with a biuret structure by continuously reacting excess amounts of organic diisocyanates having exclusively aliphatically and/or cycloaliphatically bound isocyanate groups with organic diamines having exclusively aliphatically and/or cycloaliphatically bound primary amino groups at temperatures above 170°C in the presence of acids, according to Claim 1, and isolating the polyisocyanate after the reaction, excess diisocyanate being removed from the resulting polyisocyanate solution by extraction or thin film distillation until a monomeric diisocyanate content of < 0.5 wt. % is obtained.

## Revendications

1. Procédé au diisocyanate/diamine de préparation en continu de polyisocyanates à groupement biuret en faisant réagir en continu des proportions excédentaires de diisocyanates organiques comportant exclusivement des groupes isocyanate à liaison aliphatique et/ou cycloaliphatique avec des diamines organiques comportant exclusivement des groupes amino primaires à liaison aliphatique et/ou cycloaliphatique, à des températures supérieures à 170 °C, **caractérisé en ce que** des acides sont ajoutés progressivement lors de la réaction et qu'aucun groupe isocyanate préparé par phosgénation à partir de groupes amino n'est retransformé en groupe amino.

2. Procédé selon la revendication 1, **caractérisé en ce que** sont ajoutés progressivement lors de la réaction, des acides sélectionnés dans le groupe constitué des acides phosphoriques, sulfoniques et carboxyliques.

3. Procédé selon la revendication 1, **caractérisé en ce que** sont ajoutés progressivement lors de la réaction, des esters dialkyliques de l'acide phosphorique.

4. Procédé selon la revendication 1, **caractérisé en ce que** les acides ajoutés progressivement lors de la réaction du diisocyanate avec la diamine sont mis en oeuvre en proportions de 0,01 à 1,0 % en poids par rapport au diisocyanate utilisé.

5. Procédé de préparation en continu de polyisocyanates à groupement biuret selon la revendication 1, **caractérisé en ce que** les acides mis en oeuvre lors de la réaction du diisocyanate avec la diamine sont ajoutés au diisocyanate directement avant l'adjonction dosée de la diamine.

6. Procédé de préparation en continu de polyisocyanates à groupement biuret selon la revendication 1, en faisant réagir en continu des proportions excédentaires de HDI avec de la HDA à des températures supérieures à 170 °C, **caractérisé en ce que** des acides sont ajoutés progressivement lors de la réaction.

7. Procédé de préparation en continu de polyisocyanates à groupement biuret selon la revendication 1, en faisant réagir en continu des proportions excédentaires de HDI avec de la HDA à des températures supérieures à 200 °C, **caractérisé en ce que** des acides sont ajoutés progressivement lors de la réaction.

8. Procédé de préparation en continu de polyisocyanates à groupement biuret en faisant réagir en continu des proportions excédentaires de diisocyanates organiques comportant exclusivement des groupes isocyanate à liaison aliphatique et/ou cycloaliphatique avec des diamines organiques comportant exclusivement des groupes amino primaires à liaison aliphatique et/ou cycloaliphatique, à des températures supérieures à 170 °C en présence d'acides, selon la revendication 1, et en isolant le polyisocyanate après la réaction, la solution de polyisocyanate obtenue étant débarrassée du diisocyanate excédentaire par extraction ou distillation en couche mince à des teneurs de diisocyanate monomère inférieures à 0,5% en poids.
